# EUROPÄISCHE PATENTSCHRIFT

(11) **EP 1 263 381 B1**
(45) Veröffentlichungstag und Bekanntmachung des Hinweises auf die Patenterteilung: **05.11.2003**
(21) Anmeldenummer: 01911375.2
(22) Anmeldetag: 22.01.2001
(51) Int. Cl.: A61F 13/84

(54) **DAMENSLIPEINLAGE, INSBESONDERE FÜR SCHWANGERE**
PANTY-LINER FOR WOMEN, IN PARTICULAR FOR PREGNANT WOMEN
PROTEGE-SLIP POUR FEMMES, EN PARTICULIER POUR FEMMES ENCEINTES

(30) Priorität: 24.01.2000 DE 20001141 U
(43) Veröffentlichungstag der Anmeldung: 11.12.2002
(73) Patentinhaber: Buzluhan, Hülya, 90427 Nürnberg (DE)
(72) Erfinder: Buzluhan, Hülya, 90427 Nürnberg (DE)
(74) Vertreter: Lösch, Christoph
(86) Internationale Anmeldenummer: DE0100251
(87) Internationale Veröffentlichungsnummer: WO01054642

(56) Entgegenhaltungen:
- WO-A-00/04822
- WO-A-94/24557
- DE-A- 3 035 902
- DE-U- 29 514 562
- GB-A- 2 022 423
- US-A- 5 823 953
- US-A- 5 876 389

## Beschreibung

Die Erfindung betrifft eine Damenslipeinlage mit den Merkmalen des Oberbegriffs des Patentanspruchs 1.

Als Stand der Technik ist es z.B. bekannt, Schwangere regelmäßig medizinisch im Vaginalbereich zu untersuchen und die dort auftretenden Ausscheidungen chemisch zu analysieren. In diesem Zusammenhang wird beispielsweise regelmäßig der pH-Wert im Vaginalbereich durch Verwendung von Lackmus-Teststreifen ermittelt, um z.B. einen unerwünschten vorzeitigen Blasensprung der Fruchtblase feststellen zu können, bei dem sich der pH-Wert im Vergleich zum Zustand vor dem Blasensprung spürbar verändert. Falls hierdurch ein vorzeitiger Blasensprung festgestellt wird, kann vom behandelnden Arzt eine Geburt eingeleitet werden. Wenn nun ein derartiger vorzeitiger Blasensprung nicht erkannt wird, kann dies zu gesundheitlichen Schäden für Kind und Mutter und im Extremfall zu einer Fehlgeburt führen. Die US-A-5,823,953 zeigt einen Pantyliner mit pH-Indikator, der in einer separaten Lasche abnehmbar mit dem Pantyliner verbunden ist. Die WO-A-94/24557 zeigt einen derartigen Artikel, dessen Deckschicht einteilig ausgebildet ist. Die US-A-5,876,389 zeigt eine Damenbinde, bei der eine Schicht entnommen werden kann.

Der Erfindung liegt die Aufgabe zugrunde, eine Damenslipeinlage, insbesondere für Schwangere, anzubieten, mit welcher das Risiko einer verspäteten Diagnose von Ausscheidungen im Vaginalbereich vermindert oder völlig beseitigt wird.

Diese Aufgabe wird durch die Merkmale des kennzeichnenden Teils des Patentanspruchs 1 in Verbindung mit den Merkmalen des Oberbegriffs gelöst. Vorteilhafte Ausführungsformen der Erfindung werden durch die Unteransprüche 2 - 20 realisiert.

Die erfindungsgemäße Damenslipeinlage besitzt mindestens eine integrierte mit einer Testsubstanz versehene Testeinlage über welche z.B. Ausscheidungen einer Schwangeren im Vaginalbereich und damit insbesondere ein vorzeitiger Blasensprung von der Schwangeren selbst ohne medizinische Untersuchung festgestellt werden können. Dabei befindet sich die Testeinlage unterhalb einer Deckschicht der Damenslipeinlage, so daß ein direkter Hautkontakt und mögliche Hautreizungen vermieden werden.

Bei einem vorzeitigen Blasensprung verändert sich durch das ausgeschiedene Fruchtwasser, der vaginale pH-Wert und verschiebt sich von pH 5 (Wert für vaginales Sekret) auf ca. pH 7,5 (Wert für Fruchtwasser).

Bei Verwendung von Lackmus-Papier als Testeinlage verfärbt sich dieses, so daß ein vorzeitiger Blasensprung von der Schwangeren selbst erkannt und die Schwangere möglichst umgehend einen Arzt aufsuchen kann. Ein unentdeckter vorzeitiger Blasensprung mit den geschilderten Folgen für Mutter und Kind kann dadurch vermieden werden.

Indem die Deckschicht, welche die Testeinlage überdeckt, zumindest bereichsweise transparent oder durchscheinend ausgebildet ist, kann eine Verfärbung der Testeinlage aufgrund der aufgenommenen Ausscheidungen durch die Deckschicht hindurch erkannt werden, ohne daß die Testeinlage aus der Damenslipeinlage herausgezogen werden muß.

Wenn die Testeinlage mit mehreren verschiedenen Testsubstanzen versehen wird, kann eine mehrfache Untersuchung und Analyse der Ausscheidungen im Vaginalbereich erfolgen. Neben einem vorzeitigen Blasensprung durch eine Feststellung einer Veränderung des pH-Wertes kann damit auch eine Veränderung z.B. der Zucker- oder Einweißanteile im Urin festgestellt werden.

Bei Verwendung einer einzigen Testeinlage kann diese Bereiche aufweisen, die mit verschiedenen Testsubstanzen versehen sind. Diese Bereiche können streifenförmig angeordnet sein, wobei sich zwischen den Bereichen mit den verschiedenen Testsubstanzen auch Bereiche der Testeinlage ohne Testsubstanz befinden können. Ferner können mehrere Testeinlagen, die jeweils mit einer bestimmten Testsubstanz versehen sind, verwendet werden.

Gemäß einer weiteren vorteilhaften Ausführungsform ist die Unterschicht und/oder die Deckschicht der Damenslipeinlage mit einer Öffnung versehen, über welche die Teststreifeneinlage eingeführt und zwischen Deckschicht und Unterschicht positioniert werden kann. Diese Öffnung kann z.B. schlitzförmig ausgestaltet sein. Ferner können mehrere Öffnungen vorgesehen sein.

Alternativ oder ergänzend kann die Unterschicht und/oder die Deckschicht der Damenslipeinlage zweiteilig ausgebildet sein und z.B. Teilbereiche aufweisen, welche sich überlappen. Durch Auffalten der Teilbereiche kann damit das Innere der Damenslipeinlage freigelegt und eine Testeinlage eingelegt werden. Danach werden die beiden Teilbereiche wieder geschlossen.

Vorteilhafterweise überlappen sich derartige Teilbereiche und können auch durch eine Klebeschicht und/oder durch eine Klettverbindung lösbar und positionsfixiert miteinander verbunden werden.

Um die eingelegte Testeinlage durch die Deckschicht hindurch nochmals verbessert erkennen und um deren Verfärbungen feststellen zu können, kann es vorteilhaft sein, in der Deckschicht Zonen erhöhter Durchsichtigkeit/Transparenz anzuordnen. Ferner können Zonen erhöhter Feuchtigkeitsdurchlässigkeit vorgesehen sein, um ein Ansprechen der Testsubstanz mit hoher Sicherheit zu erreichen.

Wenn die Damenslipeinlage aus einem waschbaren und wiederverwendbarem Material besteht, kann sie ressourcenschonend wiederholt gebraucht werden.

Hierzu kann die Damenslipeinlage z.B. aus Baumwolle oder einem Baumwollmischgewebe bestehen. Bei einer derartigen mehrfach wiederverwendbaren Damenslipeinlage kann nach jeder Benutzung die jeweilige Testeinlage entfernt und auf eine Verfärbung hin untersucht werden, um nach dem Waschen der Damenslipeinlage eine ungebrauchte Testeinlage in die Damenslipeinlage einzuführen, um die medizinische Überwachung fortsetzen zu können.

Ein unerwünschtes und den Tragekomfort beeinträchtigendes Verrutschen oder Verschieben der Testeinlage im Inneren der Damenslipeinlage kann dadurch verhindert werden, daß die Testeinlage im Inneren der Damenslipeinlage fixiert wird. Dies kann z.B. durch Vernähen, Verpressen und/oder Verkleben geschehen. Dabei kann die Testeinlage z.B. in Randbereichen der Damenslipeinlage zwischen der Unterschicht und der Deckschicht ein- oder zweiseitig befestigt sein.

Die Erfindung ist anhand von Ausführungsbeispielen in den schematischen Zeichnungsfiguren näher erläutert. Es zeigen:
- Fig. 1: eine Draufsicht auf eine erfindungsgemäße Damenslipeinlage,
- Fig. 2: ein Schnitt A-A nach Fig. 1,
- Fig. 3: eine Draufsicht auf eine Teststreifeneinlage,
- Fig. 4: ein Schnitt B-B nach Fig. 1,
- Fig. 5: ein Schnitt durch eine modifizierte Ausführungsform der Damenslipeinlage sowie
- Fig. 6: ein Schnitt durch eine weitere modifizierte Ausführungsform einer Damenslipeinlage.

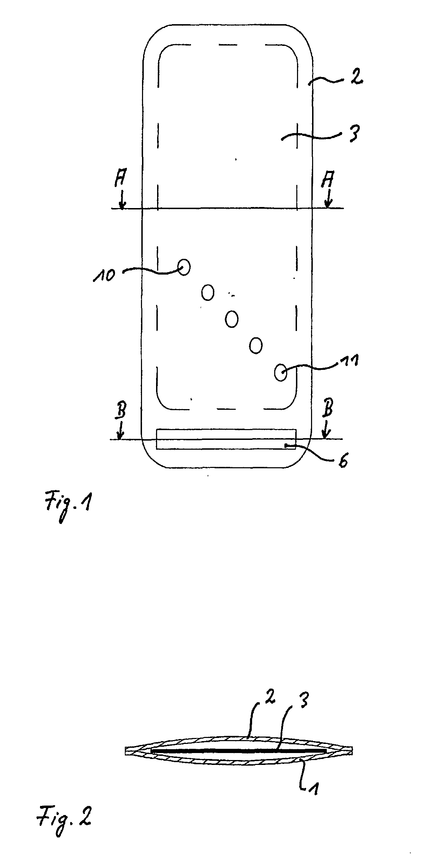

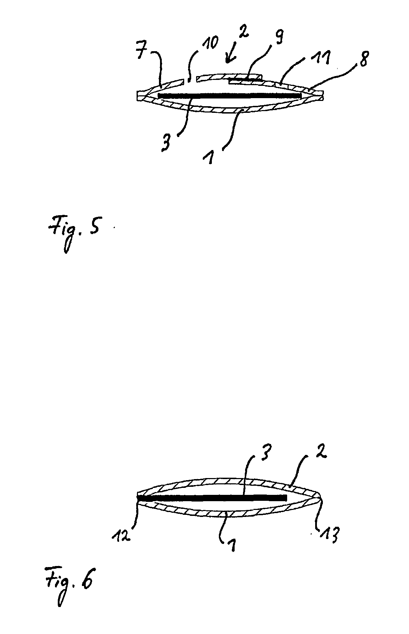

In der Draufsicht nach Fig. 1 ist die erfindungsgemäße Damenslipeinlage mit der Deckschicht 2 und der darunter angeordneten Testeinlage 3 abgebildet. Die Teststreifeneinlage 3 kann durch den Schlitz 6 eingeführt und entnommen werden. Die Deckschicht 2 weist Zonen 10, 11 erhöhter Durchsichtigkeit oder Feuchtigkeitsdurchlässigkeit auf (vgl. Fig. 5). Diese Zonen 10, 11 können auch als Ausnehmungen gestaltet sein, die insbesondere kleinflächig, z.B. schlitzförmig oder punktförmig, ausgebildet sind, um den direkten Hautkontakt mit der Testeinlage 3 zu minimieren.

Aus der Schnittdarstellung nach Fig. 2 geht hervor, daß die Testeinlage 3 zwischen der feuchtigkeitsdurchlässigen Deckschicht 2 und der feuchtigkeitsundurchlässigen Unterschicht 1 angeordnet ist. Ausscheidungen im Vaginalbereich durchdringen nun die Deckschicht 2 und kontaktieren die Testeinlage 3, so daß die Testsubstanz der Testeinlage 3 gegebenenfalls reagiert und sich verfärbt. Bei Verwendung einer Lackmus-Einlage als Testeinlage 3 kann das bei einem vorzeitigen Blasensprung einer Schwangeren ausgeschiedene Fruchtwasser entdeckt werden, da eine Verfärbung der Testeinlage 3 (Lackmus-Papier) durch die Veränderung des pH-Wertes im Vaginalbereich ermittelt wird.

Aufgrund der zumindest teilweisen Durchsichtigkeit der Deckschicht 2 und/oder der Unterschicht 1 kann diese Verfärbung der Testeinlage von der Schwangeren selbst erkannt werden. Alternativ oder ergänzend kann die Testeinlage 3 auch z.B. durch den im Endbereich (oder seitlich in Längsrichtung - nicht abgebildet) der Damenslipeinlage angebrachten Schlitz 6 (vgl. Fig. 1) aus der Damenslipeinlage zur besseren Erkennbarkeit entfernt werden. In die Damenslipeinlage können auch mehrere Testeinlagen 3 eingeführt werden (nicht abgebildet).

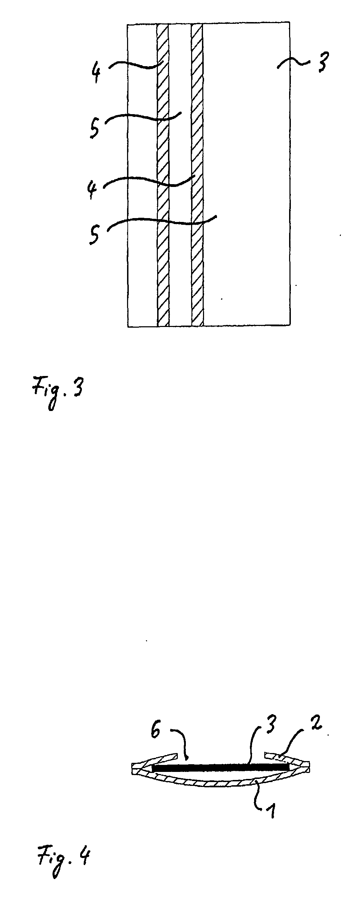

Ferner können Testeinlagen 3 gemäß Fig. 3 verwendet werden, welche erste Bereiche 4 besitzen, die mit einer Testsubstanz versehen sind sowie weitere Bereiche 5 aufweisen, in denen keine Testsubstanz eingebracht wurde. In den benachbarten und z.B. streifenförmig verlaufenden Bereichen 4 können unterschiedliche Testsubstanzen eingebracht werden, um Änderungen z.B. des pH-Wertes, des Zucker- oder Eiweißanteils erkennen zu können.

Fig. 4 zeigt eine Schnittdarstellung B-B nach Fig. 1 der Damenslipeinlage im Bereich des Schlitzes 6. In diesem Bereich kann die Testeinlage 3 zwischen die Deckschicht 2 und die Unterschicht 1 der Damenslipeinlage eingeführt oder durch Eingreifen der Schwangeren aus der Damenslipeinlage herausgezogen werden.

Gemäß einer ersten Modifikation der Damenslipeinlage nach Fig. 5 weist die Deckschicht 2 Teilbereiche 7, 8 auf, die sich überlappen und im Überlappungsbereich über eine Klebeschicht 9 (und/oder einen Klettverschluß) lösbar miteinander verbunden sind.

Wenn die Damenslipeinlage nun geöffnet werden soll, werden die Teilbereiche 7, 8 der Deckschicht 2 im Bereich der Klebeschicht 9 voneinander gelöst und die Teilbereiche 7, 8 können aufgefaltet werden, um eine Testeinlage 3 einzulegen oder zu entnehmen.

Die Teilbereiche 7, 8 weisen Zonen 10 (Ausnehmungen) und/oder 11 (Fenster) erhöhter Feuchtigkeitsdurchlässigkeit und/oder erhöhter Transparenz auf, um zuverlässig zu erreichen, daß die Ausscheidungen im Vaginalbereich auch die Testeinlage 3 erreichen und erkennbar sind. Dies kann insbesondere bei einer Damenslipeinlage gemäß Fig. 5 vorteilhaft sein, da sich die Teilbereiche 7, 8 der Deckschicht 2 überlappen und im Überlappungsbereich unter Umständen eine geringere Feuchtigkeitsdurchlässigkeit und/oder Transparenz der Deckschicht 2 auftreten kann.

In einer weiteren modifizierten Ausführungsform nach Fig. 6 der Damenslipeinlage ist die Testeinlage 3 in Randbereichen 12 und/oder 13 der Damenslipeinlage zwischen der Deckschicht 2 und der Unterschicht 1 fixiert, z.B. durch Vernähen, Verpressen und/oder Verkleben. Damit wird ein unbeabsichtigtes Verrutschen der Teststreifeneinlage 3 zwischen der Unterschicht 1 und der Deckschicht 2 vermieden.

## Patentansprüche

1. Damenslipeinlage für Schwangere, mit einer flüssigkeitsundurchlässigen Unterschicht und einer flüssigkeitsdurchlässigen Deckschicht, zwischen der Unterschicht (1) und der Deckschicht (2) eine mit einer Testsubstanz versehene Testeinlage (3) angeordnet ist,
**dadurch gekennzeichnet, daß** die Unterschicht (1) und/oder die Deckschicht (2) zweitellig ausgebildet sind und die Unterschicht (1) und/oder die Deckschicht (2) einander zumindest teilweise überlappende Teilbereiche (7, 8) aufweist.

2. Damenslipeinlage nach Anspruch 1,
**dadurch gekennzeichnet, daß** die Deckschicht (2) zur Erkennung einer Verfärbung der Testeinlage (3) zumindest teilweise transparent oder durchscheinend ist.

3. Damenslipeinlage nach Anspruch 1 oder 2,
**dadurch gekennzeichnet, daß** die Testeinlage (3) mit mindestens einer weiteren Testsubstanz versehen ist.

4. Damenslipeinlage nach einem der vorhergehenden Ansprüche,
**dadurch gekennzeichnet, daß** die Testeinlage (3) mit einer Testsubstanz zur Erkennung einer Veränderung des pH-Wertes versehen ist.

5. Damenslipeinlage nach einem der vorhergehenden Ansprüche,
**dadurch gekennzeichnet, daß** die Testeinlage (3) mindestens einen mit einer Testsubstanz versehenen ersten Bereich (4) sowie mindestens einen zweiten Bereich (5) ohne eingebrachte Testsubstanz aufweist.

6. Damenslipeinlage nach Anspruch 5,
**dadurch gekennzeichnet, daß** der erste Bereich (4) und/oder der zweite Bereich (5) im wesentlichen streifenförmig verlaufen.

7. Damenslipeinlage nach einem der vorhergehenden Ansprüche,
**dadurch gekennzeichnet, daß** die Unterschicht (1) und/oder die Deckschicht (2) mindestens eine Öffnung zur Einführung und/oder Entfernung der Testeinlage (3) aufweisen.

8. Damenslipeinlage nach Anspruch 7,
**dadurch gekennzeichnet, daß** die Öffnung als Schlitz (6) in der Unterschicht (1) und/oder in der Deckschicht (2) ausgebildet ist.

9. Damenslipeinlage nach Anspruch 1,
**dadurch gekennzeichnet, daß** die Teilbereiche (7, 8) im Bereich ihrer Überlappung lösbar miteinander verbunden sind.

10. Damenslipeinlage nach Anspruch 9,
**dadurch gekennzeichnet, daß** die Teilbereiche (7, 8) über eine Klebeschicht (9) lösbar miteinander verbunden sind.

11. Damenslipeinlage nach Anspruch 9 oder 10,
**dadurch gekennzeichnet, daß** die Teilbereiche (7, 8) über einen Klettverschluß lösbar miteinander verbunden sind.

12. Damenslipeinlage nach einem der vorhergehenden Ansprüche,
**dadurch gekennzeichnet, daß** die Deckschicht (2) Zonen (10, 11) erhöhter Feuchtigkeitsdurchlässigkeit aufweist.

13. Damenslipeinlage nach einem der vorhergehenden Ansprüche,
**dadurch gekennzeichnet, daß** die Deckschicht (2) Zonen (10, 11) mit einem erhöhten Transparenzgrad aufweist.

14. Damenslipeinlage nach einem der vorhergehenden Ansprüche,
**dadurch gekennzeichnet, daß** die Damenslipeinlage aus einem waschbaren und mehrfach wiederverwendbarem Material besteht.

15. Damenslipeinlage nach Anspruch 14,
**dadurch gekennzeichnet, daß** die Damenslipeinlage aus Baumwolle oder einem Bauwollmischgewebe besteht.

16. Damenslipeinlage nach einem der vorhergehenden Ansprüche,
**dadurch gekennzeichnet, daß** die Testeinlage (3) zwischen der Unterschicht (1) und der Deckschicht (2) fixiert angeordnet Ist

17. Damensilpeinlage nach Anspruch 16,
**dadurch gekennzeichnet, daß** die Testeinlage (3) an mindestens einem Randbereich (12, 13) mit der Unterschicht (1) und/oder der Deckschicht (2) verbunden ist

18. Damenslipeinlage nach Anspruch 16 oder 17,
**dadurch gekennzeichnet, daß** die Testeinlage (3) mit der Unterschicht (1) und/oder der Deckschicht (2) zumindest bereichsweise vernäht, verpreßt und/oder verklebt ist.

## Claims

1. Pantyliner, in particular for pregnant women, with a liquid-impermeable lower layer and a liquid-permeable covering layer, **characterized in that** a test insert (3) provided with a test substance is arranged between the lower layer (1) and the covering layer (2),
**characterized in that** the lower layer (1) and/or the covering layer (2) are formed in two sections and the lower layer (1) and/or the covering layer (2) have sections (7, 8) which at least partially overlap.

2. Pantyliner according to Claim 1,
**characterized in that** the covering layer (2) is at least partially transparent or translucent for recognizing a colour change in the test insert (3).

3. Pantyliner according to Claim 1 or 2,
**characterized in that** the test insert (3) is provided with at least one other test substance.

4. Pantyliner according to one of the preceding claims,
**characterized in that** the test insert (3) is provided with a test substance for recognizing a change in the pH.

5. Pantyliner according to one of the preceding claims,
**characterized in that** the test insert (3) has at least one first area (4) provided with a test substance, and at least one second area (5) without incorporated test substance.

6. Pantyliner according to Claim 5,
**characterized in that** the first area (4) and/or the second area (5) run essentially in a strip-like manner.

7. Pantyliner according to one of the preceding claims,
**characterized in that** the lower layer (1) and/or the covering layer (2) have at least one opening for inserting and/or removing the test insert (3).

8. Pantyliner according to Claim 7,
**characterized in that** the opening is formed as a slit (6) in the lower layer (1) and/or in the covering layer (2).

9. Pantyliner according to Claim 1,
**characterized in that** the sections (7, 8) are detachably joined to one another in the overlapping area.

10. Pantyliner according to Claim 9,
**characterized in that** the sections (7, 8) are detachably joined to one another by means of an adhesive layer (9).

11. Pantyliner according to Claim 9 or 10,
**characterized in that** the sections (7, 8) are detachably joined to one another by means of a Velcro fastening.

12. Pantyliner according to one of the preceding claims,
**characterized in that** the covering layer (2) has zones (10, 11) of increased moisture permeability.

13. Pantyliner according to one of the preceding claims,
**characterized in that** the covering layer (2) has zones (10, 11) with an increased degree of transparency.

14. Pantyliner according to one of the preceding claims,
**characterized in that** the pantyliner consists of a washable material which can be reused a number of times.

15. Pantyliner according to Claim 14,
**characterized in that** the pantyliner consists of cotton or a cotton blend.

16. Pantyliner according to one of the preceding claims,
**characterized in that** the test insert (3) is arranged in a fixed manner between the lower layer (1) and the covering layer (2).

17. Pantyliner according to Claim 16,
**characterized in that** the test insert (3) is joined at at least one edge region (12, 13) to the lower layer (1) and/or the covering layer (2).

18. Pantyliner according to Claim 16 or 17,
**characterized in that** the test insert (3) is sewn, pressed and/or pasted to the lower layer (1) and/or the covering layer (2) at least in some areas.

## Revendications

1. Protège-slip pour femmes enceintes, comprenant une couche inférieure imperméable aux liquides et une couche supérieure perméable aux liquides, un insert de test (3) pourvu d'une substance de test étant disposé entre la couche inférieure (1) et la couche supérieure (2),
**caractérisé en ce que** la couche inférieure (1) et/ou la couche supérieure (2) sont réalisées en deux parties et la couche inférieure (1) et/ou la couche supérieure (2) présentent au moins des régions partielles (7, 8) se chevauchant au moins partiellement.

2. Protège-slip pour femmes selon la revendication 1,
**caractérisé en ce que** la couche supérieure (2) est au moins partiellement transparente ou translucide pour permettre de reconnaître une coloration de l'insert de test (3).

3. Protège-slip pour femmes selon la revendication 1 ou 2,
**caractérisé en ce que** l'insert de test (3) est pourvu d'au moins une autre substance de test.

4. Protège-slip pour femmes selon l'une quelconque des revendications précédentes,
**caractérisé en ce que** l'insert de test (3) est pourvu d'une substance de test permettant de déceler une variation de la valeur de pH.

5. Protège-slip pour femmes selon l'une quelconque des revendications précédentes,
**caractérisé en ce que** l'insert de test (3) présente au moins une première région (4) pourvue d'une substance de test ainsi qu'au moins une deuxième région (5) sans substance de test.

6. Protège-slip pour femmes selon la revendication 5,
**caractérisé en ce que** la première région (4) et/ou la deuxième région (5) s'étendent essentiellement en forme de bande.

7. Protège-slip pour femmes selon l'une quelconque des revendications précédentes,
**caractérisé en ce que** la couche inférieure (1) et/ou la couche supérieure (2) présentent au moins une ouverture pour l'insertion et/ou le retrait de l'insert de test (3).

8. Protège-slip pour femmes selon la revendication 7,
**caractérisé en ce que** l'ouverture est réalisée sous forme de fente (6) dans la couche inférieure (1) et/ou dans la couche supérieure (2).

9. Protège-slip pour femmes selon la revendication 1,
**caractérisé en ce que** les régions partielles (7, 8) sont reliées l'une à l'autre de manière détachable dans la région de leur chevauchement.

10. Protège-slip pour femmes selon la revendication 9,
**caractérisé en ce que** les régions partielles (7, 8) sont reliées l'une à l'autre de manière détachable par le biais d'une couche de colle (9).

11. Protège-slip pour femmes selon la revendication 9 ou 10,
**caractérisé en ce que** les régions partielles (7, 8) sont reliées l'une à l'autre de manière détachable par le biais d'une fermeture velcro.

12. Protège-slip pour femmes selon l'une quelconque des revendications précédentes,
**caractérisé en ce que** la couche supérieure (2) présente des zones (10, 11) de perméabilité à l'humidité accrue.

13. Protège-slip pour femmes selon l'une quelconque des revendications précédentes,
**caractérisé en ce que** la couche supérieure (2) présentent des zones (10, 11) de transparence accrue.

14. Protège-slip pour femmes selon l'une quelconque des revendications précédentes,
**caractérisé en ce que** le protège-slip pour femmes se compose d'un matériau lavable et réutilisable plusieurs fois.

15. Protège-slip pour femmes selon la revendication 14,
**caractérisé en ce que** le protège-slip pour femmes se compose de coton ou d'un tissu mixte contenant du coton.

16. Protège-slip pour femmes selon l'une quelconque des revendications précédentes,
**caractérisé en ce que** l'insert de test (3) est disposé de manière fixe entre la couche inférieure (1) et la couche supérieure (2).

17. Protège-slip pour femmes selon la revendication 16,
**caractérisé en ce que** l'insert de test (3) est connecté au niveau d'au moins une région de bord (12, 13) à la couche inférieure (1) et/ou à la couche supérieure (2).

18. Protège-slip pour femmes selon la revendication 16 ou 17,
**caractérisé en ce que** l'insert de test (3) est cousu, pressé et/ou collé au moins partiellement à la couche inférieure (1) et/ou à la couche supérieure (2).
